# EUROPEAN PATENT APPLICATION

(11) **EP 4 650 775 A1**
(43) Date of publication of application: **19.11.2025**
(21) Application number: 24175582.6
(22) Date of filing: 14.05.2024
(51) Int. Cl.: G01N 33/533, G01N 33/58, C07K 7/00, C07K 14/00

(54) **SMALL BRIGHT FLUORESCENT LABELS BASED ON PEPTIDIDE BACKBONE WITH HIGH TISSUE PENETRATION**

(71) Applicant: Miltenyi Biotec B.V. & Co. KG, 51429 Bergisch Gladbach (DE)
(72) Inventor: DOSE, Christian, D-51429 Bergisch Gladbach (DE); FAUERBACH, Jonathan, 51429 Bergisch Gladbach (DE); REIBER, Thorge, 51429 Bergisch Gladbach (DE); YUSHCHENKO, Dmytro, 51429 Bergisch Gladbach (DE)
(74) Representative: Kisters, Michael Marcus

(57) **Abstract**

The invention is directed to fluorescent probes comprising an antigen recognizing moiety covalently bound by a peptide backbone to at least one fluorescent dye **characterized in that** the fluorescent probes have the general formula I

Y-(L-(A(F)-Pi)ₙ-A(F)-P')ₘ

With
Pi: same or different peptide comprising 10 - 30 amino acids,
P': a peptide comprising 0 - 50 amino acids
F: fluorescent moiety
A: an amino acid,
L: a linker unit
Y: antigen or antigen recognizing moiety
m: 1-100

## Description

### BACKGROUND

This invention relates to dyes for fluorescence microscopy.

Fluorescence microscopy is a powerful technique used to generate high-content imaging datasets for investigating cell biology and developing therapies. Particularly super-resolution and 3D light sheet microscopy became important in the last years as they enabled to push the boundaries of microscopy from single molecule level to a whole organ respectively. Both methods depend on the fluorescent labels that determine the quality of epitope staining and the quality of imaging. The ideal label would be on the one hand small, to efficiently and specifically label epitopes, on the other hand bright, to collect high number of photons to get a high-quality image.

A small size of a label would allow precise epitope localization, which is the main required feature in super-resolution imaging and related applications. Small label size may additionally increase tissue penetration, that is crucial for proper labeling and successful imaging of whole organs or organ sections in 3D microscopy. High brightness of the labels in combination with specific staining determines the signal-to-noise ratio, which is one of the most important quantitative characteristics of a fluorescence image.

Unfortunately, development of small labels with high brightness is not a trivial task. The fluorescent label is typically composed of at least two functionalities: a binder and a fluorescent moiety. Even if a binder is relatively small but the bright fluorescent moiety is bulky they would form a large label.

### SUMMARY

To address the aforementioned limitations, we designed novel labels based on peptides as small binders in conjunction with an optimal size of the fluorescent moiety to minimize dye-dye interactions, which usually results in self-quenching and lowers the fluorescent signal.

It was found that self-quenching can be avoided by providing fluorescent moieties to a peptide backbone in a way that the fluorescent moieties have a sufficient distance to each other.

Accordingly, object of the invention are fluorescent probes comprising an antigen recognizing moiety covalently bound by a peptide backbone to at least one fluorescent dye **characterized in that** the fluorescent probes have the general formula I

Y-(L-(A(F)-Pi)ₙ-A(F)-P')ₘ

With
Pi: same or different peptide comprising 10 - 30 amino acids,
P': a peptide comprising 0 - 50 amino acids
F: fluorescent moiety
A: an amino acid,
L: a linker unit
Y: antigen or antigen recognizing moiety
m: 1-100

Pi stands (for n>1) for same or different peptides, each comprising 10 - 30 amino acids, i.e. each Pi peptide may have the same or a different amino acid sequence for every n.

The fluorescent probes of the present invention are especially useful for fluorescence microscopy of cells and tissues, for example in for light-sheet, ultra microscopy and other applications that require tissue penetration and/or for super-resolution microscopy applications (STED, PALM, STORM, MINFLUX, etc) for imaging objects smaller than 100 nm and/or for microscopy application that require high density of epitope labeling and/or imaging of samples with poor accessibility of epitopes.

Further, the fluorescent probes may be used in high content imaging, e.g. with MICS (MACSima Imaging Cyclic Staining) technology and/or for conventional and spectral flow cytometry applications.

Our results show that labels developed based on this approach provide specific staining and overperform conventional labels especially for the penetration of tissue. E.g., multimerization of the dye Cy5 on peptide backbones enables more robust and reliable detection of cells in light sheet microscopy when performed with nanobodies as binders. In contrast, nanobodies labelled with conventional NHS- and maleimide-based dyes show overall lower brightness and a more rapid decline in emission when moving in deeper tissue. In addition, time of tissue staining with the proposed labels is manyfold reduced due to faster tissue penetration abilities of nanobodies decorated with fluorescent peptides.

### BRIEF DESCRIPTION OF THE DRAWINGS

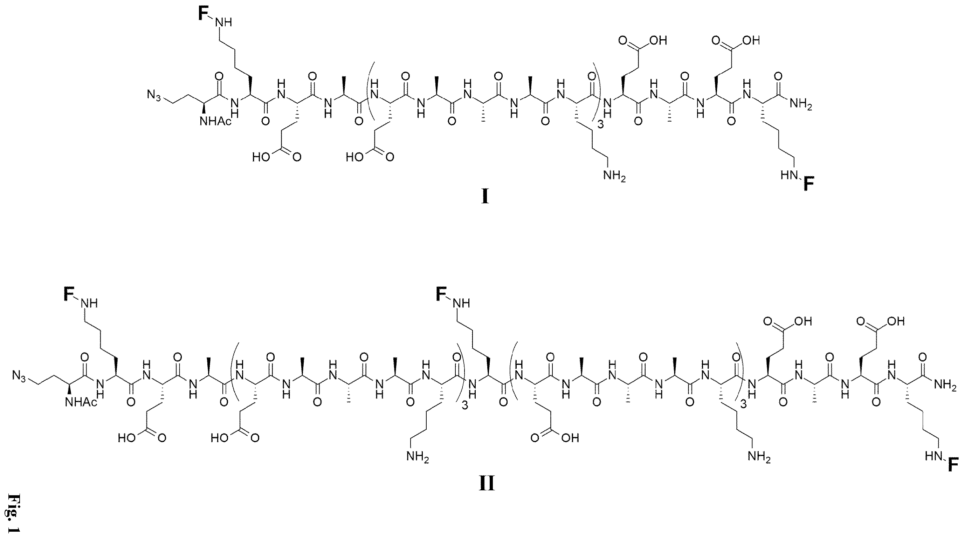

Fig. 1 shows the chemical structures of peptide-based conjugates Y-L-I and Y-L-II for fluorescence applications. F represent fluorophores bound to lysine.

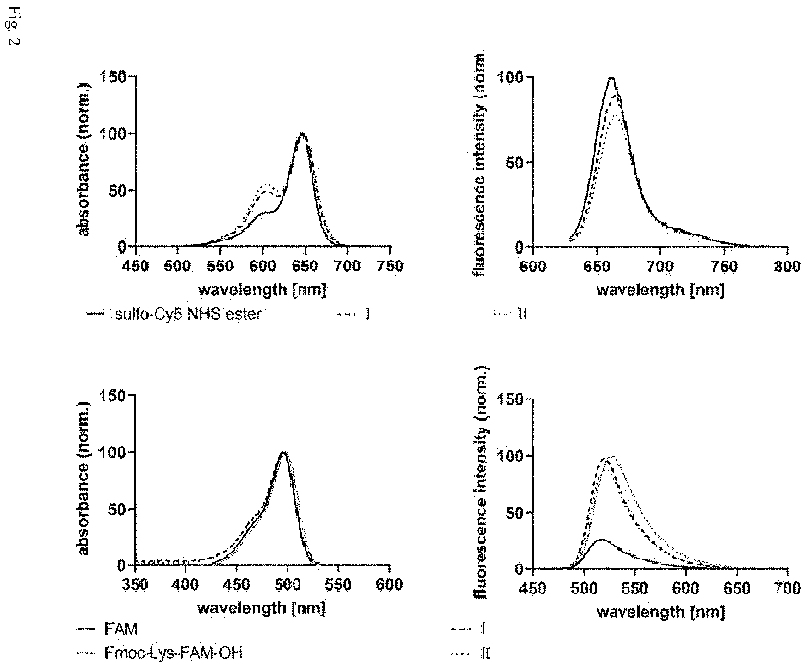

Fig. 2 shows the absorption and emission spectra of monomeric dye Cy5, FAM and Fmoc-Lys-FAM-OH, respectively, as well as peptide-based conjugates. Peptide-dye compounds do not have significant fluorescence quenching and thus loss of brightness.

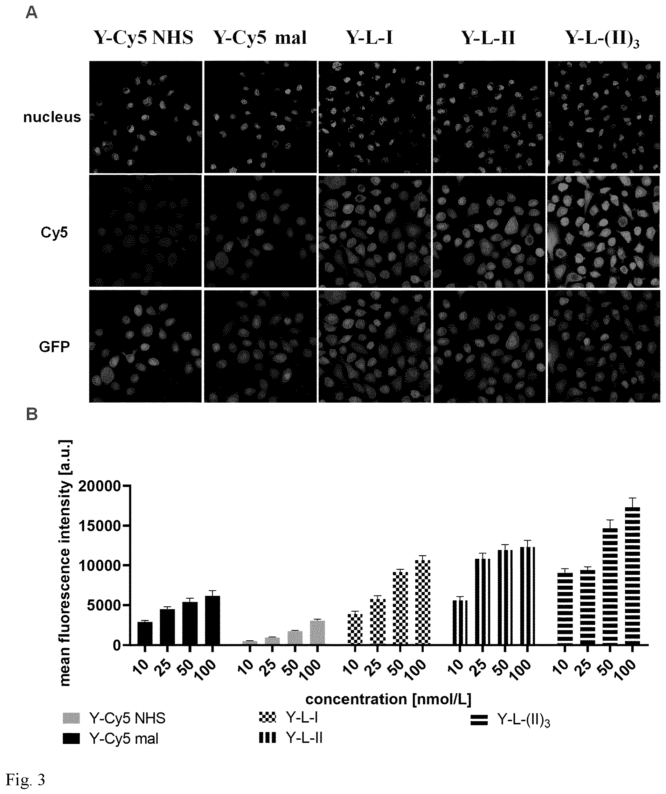

Fig. 3 shows a comparison of performance of peptide-nanobody conjugates (Y-L-I Y-L-II & Y-L-(II)₃) with the performance of respective conventional probes (Y-Cy5 mal & Y-Cy5 NHS) in confocal fluorescence microscopy. (A) Confocal images of AsPC-1 cells expressing cytosolic GFP after their fixation and staining with DAPI and conventional Cy5 nanobodies against GFP (Y-Cy5 NHS and Y-Cy5 mal) or Cy5 of peptide-nanobody conjugates (Y-L-I Y-L-II & Y-L-(II)₃) respectively. First row: nucleus staining (DAPI signal); Middle row: staining with anti-GFP nanobody conjugates (Cy5 signal); Last row: native GFP signal. (B) Quantitative analysis of Cy5 signal, where MFI of conjugates is evaluated and compared at different conjugate concentrations. Peptide-based probes show higher fluorescence intensity on cells in microcopy experiments when compared to conventional conjugates.

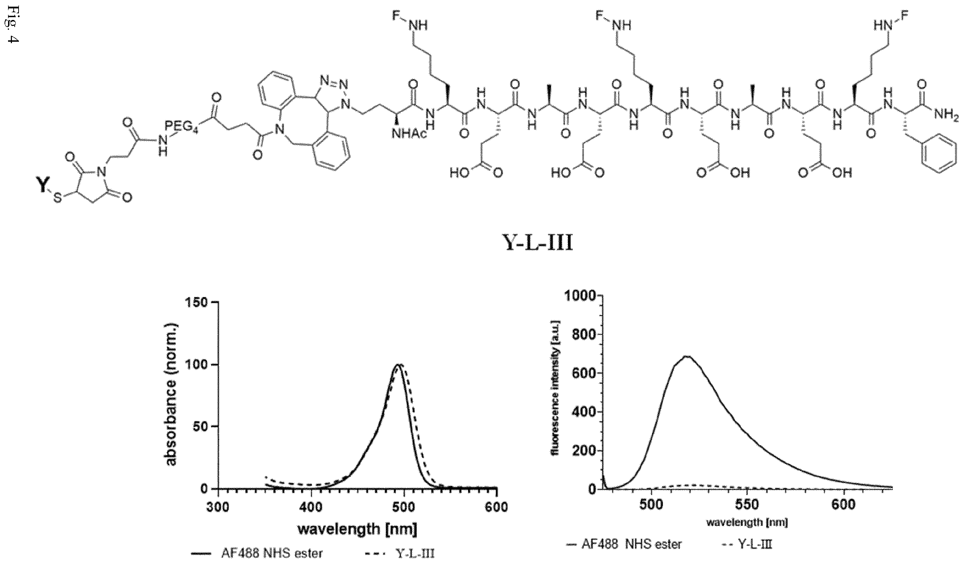

Fig. 4 shows structure and photospectroscopy of peptide conjugate Y-L-III with similar backbone, however, with a structure not according to the invention. Fluorescence spectra of conjugates show decreased fluorescence intensity when compared with the monomeric dye, demonstrating the insufficient distance between two adjacent fluorophores leading to their self-quenching. AF488 is among fluorophores listed in claims and was used as a reference.

### DETAILED DISCLOSURE

The probes of the invention have in general formula (I), i.e. Y-(L-(A(F)-Pi)ₙ-A(F)-P')ₘ. In certain embodiments of the invention, P' stand for a direct bond, i.e. contain no amino acids. In other words, the probes of the invention may have general formula (II), i.e.

Y-(L-(A(F)-Pi)ₙ-A(F))ₘ (II)

With
Pi: same or different peptide comprising 10 - 30 amino acids,
F: fluorescent moiety
A: an amino acid,
L: a linker unit
Y: antigen or antigen recognizing moiety
m: 1-100

As for formula (I), Pi stands (for n>1) for same or different peptides, each comprising 10 - 30 amino acids, i.e. each Pi peptide may have the same or a different amino acid sequence for every n.

The linker unit L may be selected from the group consisting of peptides having 1 - 50 amino acids, DBCO, oligoglycols, polyethyleneglycols (PEG), polysaccharide.

The antigen recognizing moiety Y may be any unit binding to antigens presented on the surface of cells, and are preferable selected from the group comprising proteins, peptides antibodies, antibody fragments, nanobodies, polysaccharides, lipids, nucleic acids.

The fluorescent moiety F may selected from any group known to the person skilled in the art, especially form the group comprising FAM, Cy5, dyes based on xanthene, cyanine, squaraine, napthalene, coumarin, pyrene, BODIPYs core.

Suitable fluorescent moieties F are available under tradenames like Vio or Alexa Fuor dyes.

In general, amino acid A may by any natural or unnatural (artificial) amino acid known to the person skilled in the art, but preferred is Lysin.

Further, amino acid A may be provided with a PEG or an aliphatic chain as linker unit binding to the fluorescent moiety F.

### EXAMPLES

### Peptide synthesis: Automatic Solid-Phase Peptide Synthesis

Automated peptide synthesis was performed with an Alstra Initiator+ from *Biotage* (Uppsala, Sweden). All reagents were dissolved in DMF if not stated otherwise. DIPEA was mixed with NMP.

Peptides were synthesized with Tentagel R RAM resin from *Rapp Polymere GmbH* (Tübingen, Germany) with a loading of 0.18 mmol/g and a scale of 60 µmol. Reaction was carried out in a 10 mL reactor vial with PTFE frit. Amino acids were dissolved at 0.3 mol/L. Prior to starting the synthesis, the resin was swollen in DMF for 5 min at 50 °C. Cycles are given below:
Amino acid 1: Swelling of resin à Fmoc-deprotection à coupling à washing à capping
Amino acids 2 - X: Fmoc-deprotection à coupling à washing à capping
Final amino acid: Fmoc-deprotection à coupling à washing à capping à fmoc-deprotection à capping à pre-cleavage wash

By way of example, two probes (referred to as compound I and II) have been synthesized having the formulas as shown in Fig. 1 and the following sequences:
Sequence of compound I:
   Aha-Lys-Glu-Ala-(Glu-Ala-Ala-Ala-Lys)₃-Glu-Ala-Glu-Lys
   In regard to formula (I), Y-(L-(A(F)-Pi)ₙ-A(F)-P')ₘ, compound I can be described as follows
   P'= no amino acids; P1= Glu-Ala-(Glu-Ala-Ala-Ala-Lys)₃-Glu-Ala-Glu, [i=1];
   A = Lysine; and m=1 (linker unit L and antigen or antigen recognizing moiety Y omitted)
Sequence of compound II:
   Aha-Lys-Glu-Ala-(Glu-Ala-Ala-Ala-Lys)₃-Lys-(Glu-Ala-Ala-Ala-Lys)₃-Glu-Ala-Glu-Lys
   In regard to formula (I), Y-(L-(A(F)-Pi)ₙ-A(F)-P')ₘ, compound II can be described as follows
   P'= no amino acids; P1= Glu-Ala-(Glu-Ala-Ala-Ala-Lys)₃, [i=1, 17 amino acids]; P2= Glu-Ala-(Glu-Ala-Ala-Ala-Lys)₃-Glu-Ala-Glu, [i=2, 20 amino acids];
   A = Lysine; and m=1 (linker unit L and antigen or antigen recognizing moiety Y omitted)

### Amino acids used for synthesis:

Aha: Fmoc-azidohomoalanine-OH
Lys: Fmoc-Lys(Alloc)-OH
Glu: Fmoc-glutamic acid(OtBu)-OH
Ala: Fmoc-alanine-OH

### Cycle methods during solid-phase peptide synthesis:

Fmoc-deprotection: 20% piperidine in DMF (100 eq.), 1 × 3 min room temperature and 1 × 5 min at room temperature
Coupling: Amino acids (7.5 eq.) were activated with OxymaPure (7.5 eq., 0.5 M) and DIC (7.5 eq., 0.5 M) and coupled for 5 min at 75 °C
Washing steps: 3 × DMF
Capping: Capping was done after each coupling round and the last deprotection with acetic anhydride (83 eq., 5 M) and DIPEA (83 eq., 2 M in NMP) for 5 min at room temperature.
Pre-cleavage wash: 3 × DCM

### Alloc deprotection:

The resin was swollen in DCM (5 mL) for 1 h at room temperature in the SPPS reaction vial. Next, the DCM was removed by filtration and a mixture of Pd(PPh₃)₄ (0.25 eq.) and triphenyl silane (15 eq.) in argon-purged DCM (2 mL) was added to the resin and the reactor vial was transferred to a 100 mL argon-flooded three-necked flask. The mixture was stirred for 20 min under argon at room temperature and the solvent removed by filtration. The resin was washed with DCM (3 x 5 mL) and the procedure repeated. To validate the success of the reaction, a few milligrams of resin were treated with cleavage cocktail and the peptide analyzed in LC/MS.

### Fluorescent labeling of peptides on solid support:

The respective NHS-activated fluorophore (5 mg, 2.5 eq. per lysine) were dissolved in DMF (200 µL) and added to the appropriate amount of resin-bound peptide (1 eq.) suspended in DMF (200 µL). The mixture was treated with DIPEA (50 eq.) and stirred for 16 h at 40 °C in the dark. The resin was washed with DMF (3 x 5 mL) and DCM (3 x 5 mL) and dried *in vacuo.* Cleavage and purification was performed according to the general procedure I.

### Cleavage from the solid support:

Peptides were cleaved from the resin after drying in vacuo with 3 mL of a mixture of TFA/H₂O/TIPS (95/2.5/2.5, v/v/v) and phenol (1 w%) at room temperature for 90 min under exclusion of light in the same vessel that was used for synthesis. The mixture was filtrated and the resin washed with TFA (3 x 1 mL). During filtration and wash, the filtrate was added dropwise to ice-cold diethyl ether (10 mL) in 50 mL glass Eppendorf falcons and stored at - 20 °C over night. The precipitate was collected by centrifugation (1000 g, 5 min) and decantation of solvent. The precipitate was air-dried for 1 h under exclusion of light, dissolved in aqueous acetonitrile (50%, 0.1% TFA) and purified via HPLC.

### Bioconjugation to protein binders:

### Site-directed DBCO-modification of antibodies and nanobodies:

The respective protein (5 mg/mL for antibodies, 2 mg/mL for nanobodies, 1 eq.) in PBS was treated with TCEP (25 mg/mL, 5 eq. for antibodies, 1.5 eq. for nanobodies) and reacted for 30 min at room temperature. Subsequently, the respective linker (20 mg/mL, 5 eq,) in DMSO were directly added to the reduced protein binder. The reaction was further left on the shaker (1000 rpm) for 2 h at room temperature before purified by Amicon filtration (5x wash) with PBS. Antibodies and nanobodies against various epitopes available on/in cells can be modified using this method, e.g. aEGFR, aCD4, aCD3, aEpCAM, aGFP, etc.

### Click reaction of protein binders with azido-peptides:

The respective DBCO-functionalized protein binder (2.5 mg/mL for antibodies, 0.5 mg/mL for nanobodies) was treated with the appropriate amount of azido-peptides (2 mg/mL, 5 eq.) dissolved in DMSO. To prevent protein degradation, DMSO content was kept below 5 vol%. The reaction mixture was left on the shaker for 4 h at room temperature under exclusion of light. The product was purified either by NAP column or on the ÄKTA system by SEC.

### Confocal Laser-Scanning Fluorescence Microscopy:

Stained samples were imaged on a Zeiss LSM 710 confocal microscope from Carl Zeiss AG (Oberkochen, Germany). Conjugates were excited with the Ar-laser (488 nm) or HeNe2 laser (543 nm, 633 nm) and the images taken with a "Plan-Apochromat" 40x/0,95 Korr M27 objective. Laser power and gain were adjusted based on the brightest sample and kept constant for each conjugate with the same fluorophore throughout the experiment. Images were saved as .lsm5 file.

### Staining procedure for fixed and live cells in suspension:

Cells in medium were centrifuged (300g, 5 min), washed with PBS once, resuspended in PBS, and stored at 4 °C prior to fixation. Cells were fixed for 15 min in 3.7% formaldehyde at room temperature. PFA was removed, cells were washed once with PBS buffer, resuspended and stored at 4 °C. Cells were permeabilized with Inside Perm for 20 min and washed with PBS. Conjugates were adjusted to an appropriate concentration in PBS and incubated with cells for 10 min at room temperature in the dark resulting in the desired final conjugate concentrations. Cells were centrifuged (2000 rpm, 5 min), staining solution was removed, cells were washed again, and resuspended in 300 µL PBS. Cells were then centrifuged (300g, 5 min) and imaged.

### Comparative examples (not according to the invention)

Compound Y-L-III was prepared similarly to Y-L-I and Y-L-II with the difference that the length of Pi is lower than 10 amino acids.

### Sequence of compound III:

Aha-Lys-Glu-Ala-Glu-Lys-Glu-Ala-Glu-Lys-Phe
In regard to formula (I), Y-(L-(A(F)-Pi)ₙ-A(F)-P')ₘ, the comparative compound can be described as follows
P'= Phe; P1= Glu-Ala-Glu [i=1 with P comprising 3 amino acids]; P2= Glu-Ala-Glu [i=2, with P comprising 3 amino acids]
A = Lysine; m=1

Fig. 2 shows the absorption and emission spectra of dyes according to the invention comprising different fluorescent moieties F, namely the commercial available dyes CyS, FAM and Fmoc-Lys-FAM-OH compared to the absorption and emission spectra of the respective monomeric dyes (i.e. dyes not bound to peptide structures according to the invention). The absorption and emission spectra of the dyes according to the invention and the monomeric dyes are very similar, i.e. the peptide structure does not significantly interfere with the absorption and emission properties of the dyes compared to their free monomeric state.

In comparison, Fig. 4 shows the absorption and emission spectra of a commercially available dye (AF 488 NHS ester) in monomeric form and bound to a peptide according to formula III, i.e. not according to the invention. Fig. 4 exhibits a 25-fold decrease in fluorescence comparing the monomeric form and the peptide conjugate.

This strongly supports the ability of compounds according to the invention like compounds I and II to retain fluorescence properties in a peptide conjugate by minimizing dye-dye interactions. The peptide-dye compounds of the invention do not have significant fluorescence quenching and thus loss of brightness and are therefore suitable as novel fluorescence probes for fluorescence microscopy of cells and tissues.

## Claims

1. Fluorescent probes comprising an antigen recognizing moiety covalently bound by a peptide backbone to at least one fluorescent dye **characterized in that** the fluorescent probes have the general formula I
Y-(L-(A(F)-Pi)ₙ-A(F)-P')ₘ
With
Pi: same or different peptide comprising 10 - 30 amino acids,
P': a peptide comprising 0 - 50 amino acids
F: fluorescent moiety
A: an amino acid,
L: a linker unit
Y: antigen or antigen recognizing moiety
m: 1-100

2. Fluorescent probe according to claim 1 **characterized in that** the linker unit L is selected from the group consisting of peptides having 1 - 50 amino acids, DBCO, oligoglycols, PEG and polysaccharides.

3. Fluorescent probe according to claim 1 or 2 **characterized in that** the antigen recognizing moiety Y is selected from the group comprising proteins, peptides, antibodies, antibody fragments, nanobodies, polysaccharides, lipids, nucleic acids.

4. Fluorescent probe according to any of claims 1 to 3 **characterized in that** the fluorescent moiety F is selected from the group comprising FAM, CyS, dyes based on xanthene, cyanine, squaraine, napthalene, coumarin, pyrene, BODIPYs core.

5. Fluorescent probe according to any of claims 1 to 4 **characterized in that** the amino acid A is Lysin.

6. Fluorescent probe according to any of claims 1 to 5 **characterized in that** the amino acid A is provided with a PEG or an aliphatic chain as linker unit binding to the fluorescent moiety F.

7. Fluorescent probe according to any of claims 1 to 6 **characterized in that** the fluorescent probes have the general formula II
Y-(L-(A(F)-Pi)ₙ-A(F))ₘ (II)
With
Pi: same or different peptide comprising 10 - 30 amino acids,
F: fluorescent moiety
A: an amino acid,
L: a linker unit
Y: antigen or antigen recognizing moiety
m: 1-100

8. Use of the fluorescent probes according to any of the claims 1 to 7 for fluorescence microscopy of cells and tissues.
